# EUROPÄISCHE PATENTSCHRIFT

(11) **EP 3 130 315 B1**
(45) Veröffentlichungstag und Bekanntmachung des Hinweises auf die Patenterteilung: **11.10.2017**
(21) Anmeldenummer: 16179310.4
(22) Anmeldetag: 13.07.2016
(51) Int. Cl.: A61F 2/18

(54) **LASER-AKTIVIERBARE LÄNGENVARIABLE GEHÖRKNÖCHELCHENPROTHESE**
LASER ACTIVATABLE OSSICULAR PROSTHETIC WITH VARIABLE LENGTH
PROTHÈSE D'OSSELETS D'OREILLE À LONGUEUR VARIABLE POUVANT ÊTRE ACTIVÉE PAR LASER

(30) Priorität: 10.08.2015 DE 102015113138
(43) Veröffentlichungstag der Anmeldung: 15.02.2017
(73) Patentinhaber: Heinz Kurz GmbH Medizintechnik, 72144 Dusslingen (DE)
(72) Erfinder: McElveen Jr., John, T., Raleigh, NC 27609 (US); Steinhardt, Uwe, 72145 Hirrlingen (DE)
(74) Vertreter: Kohler Schmid Möbus Patentanwälte

(56) Entgegenhaltungen:
- WO-A1-02/069850
- WO-A2-2008/027862
- DE-B3-102009 016 468

## Beschreibung

Die Erfindung betrifft eine Gehörknöchelchenprothese, die mindestens ein Glied der menschlichen Gehörknöchelchenkette ersetzt oder überbrückt, wobei die Gehörknöchelchenprothese an ihrem einem Ende ein als perforierte Kopfplatte zur Anlage am Trommelfell ausgebildetes erstes Befestigungselement und an ihrem anderen Ende ein zweites Befestigungselement zur mechanischen Verbindung der Prothese mit dem Steigbügelkopf oder der Steigbügel-Fußplatte sowie ein die beiden Befestigungselemente Schall leitend miteinander verbindendes, längliches Verbindungselement aufweist, welches eine Verstellvorrichtung zur Einstellung der axialen Länge der Gehörknöchelchenprothese in Achsrichtung des länglichen Verbindungselements umfasst, wobei das erste Befestigungselement mit einem Ende und das zweite Befestigungselement mit dem axial entgegengesetzten anderen Ende des Verbindungselements mechanisch starr verbunden ist, wobei die Verstellvorrichtung mindestens zwei symmetrisch zur Längsachse des Verbindungselements verlaufende, in Achsrichtung streckbare und/oder stauchbare Teilstränge umfasst, die dauerhaft bleibend plastisch verformbar und vor dem Verformen quer zur Längsachse in mehreren Schlaufen Schlangenförmig, Mäanderförmig bzw. Ziehharmonika-artig gefaltet sind, wobei jeder Teilstrang mindestens drei Schlaufen umfasst, und wobei Teile der Gehörknöchelchenprothese aus einem Material mit Formgedächtnis hergestellt sind.

Eine derartige Vorrichtung ist bekannt aus der DE 10 2009 016 468 B3.

Gehörknöchelchenprothesen werden verwendet, um bei ganz oder teilweise fehlenden oder geschädigten Gehörknöchelchen des menschlichen Mittelohrs den Schall bzw. das Schallsignal vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung bzw. Signalübertragung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden am Amboss fixiert und ragen über einen Stempel (=Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Ein Hauptproblem, das bei jeder Rekonstruktion der menschlichen Gehörknöchelchenkette auftaucht, ist die Auswahl der richtigen Prothesenlänge. Anatomisch bedingt, variieren die jeweils erforderlichen Längen in einem Spektrum von mehreren Millimetern. Daher muss beim operativen Einsetzen einer Gehörknöchelchenprothese entweder eine ausreichend große Auswahl von Prothesen unterschiedlicher axialer Länge bereitgehalten werden oder die verwendeten Gehörknöchelchenprothesen müssen während der Operation ausgehend von einer maximalen Ausgangslänge auf die erforderliche axiale Endlänge gebracht werden können.

Vorteilhaft im Hinblick auf eine postoperative Lageanpassung sind Gehörknöchelchenprothesen, bei die ganz oder teilweise, insbesondere im Bereich eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist. Dieser Effekt wird beispielsweise auch bei einer Gehörknöchelchenprothese genutzt, wie sie in der DE 10 2007 008 851 B3 beschrieben ist. Dabei wird eine anfänglich halboffene Schlinge, die während der Implantation als Befestigungselement etwa über den Amboss oder den Hammergriff gezogen wird, quasi berührungslos um das entsprechende Gehörknöchelchen geschlossen, so dass die Prothese daran fixiert ist. In ähnlichem Zusammenhang beschreibt etwa die US 6,197,060 B1 eine Artikel Selbstsicherung durch eine thermische Aktivierung eines Prothesenabschnitts mittels Laser.

Eine mit Hilfe von Klemmwirkung längenverstellbare Anordnung zum mechanischen Ankoppeln des Treibers eines aktiven Hörgeräts an eine Ankoppelstelle der Gehörknöchelchenkette ist in der DE 199 48 375 A1 beschrieben.

In der WO 92/18066 A1 ist eine selbstanpassende passive Gehörknöchelchenprothese beschrieben, die einen komplizierten und in der Herstellung sehr aufwändigen Federmechanismus in der Verbindung zwischen dem ersten und dem zweiten Befestigungselement aufweist, welcher eine ständige Änderung der axialen Länge der Prothese je nach relativer Lage der Befestigungspunkte im Mittelohr bewirkt. Eine reproduzierbar exakte, feste Längeneinstellung der Prothese, die auch nach dem operativen Einsetzen derselben ins Mittelohr erhalten bleibt, ist damit nicht möglich. Zudem erfordert die bekannte Prothese wegen ihres sehr speziellen mechanischen und geometrischen Aufbaus einen erheblichen Platzbedarf im Mittelohr, so dass sie in vielen Fällen aufgrund der individuellen Gegebenheiten beim Patienten gar nicht einsetzbar ist. Außerdem wird Konstruktionsbedingt nach dem Einsetzen ein nicht unerheblicher permanenter Druck zwischen den beiden Befestigungspunkten im Mittelohr aufgebaut, was einer Heilung nach der Operation nicht gerade förderlich ist und auf Dauer häufig zu postoperativen Komplikationen führt.

Eine passive Gehörknöchelchenprothese mit während der Operation in bestimmten Grenzen variierbarer axialer Länge ist in der DE 39 01 796 A1 beschrieben. Dabei wird die Längenänderung durch ein Verbiegen des als dünner aus Golddraht gefertigten Verbindungselements erreicht, was allerdings einerseits umständlich in der Handhabung, andererseits ziemlich ungenau ist, so dass damit keine exakte Einstellung der gewünschten axialen Länge der Gehörknöchelchenprothese erreicht werden kann. Außerdem ist das Ergebnis bei dieser Technik nicht immer reproduzierbar und es kann sogar vorkommen, dass sich nach dem Verbiegen des Verbindungselements die eingestellte axiale Länge der Gehörknöchelchenprothese durch ein Zurückfedern des Verbindungselements wieder ändert.

Die EP 0 998 884 A2 beschreibt eine passive Gehörknöchelchenprothese, bei welcher das als länglicher Schaft ausgebildete erste Verbindungselement durch eine Durchgangsbohrung des als Kopfplatte ausgebildeten ersten Befestigungselements hindurch gesteckt wird, bis eine gewünschte Schaftlänge zwischen dem ersten und dem zweiten Befestigungselement erreicht ist. Sodann wird der Schaft in dieser Position durch Verengen der Durchgangsbohrung in der Kopfplatte fixiert und der über die Kopfplatte hinaus überstehende Teil des Schaftes abgelängt. So erhält man auf einfache Weise eine Prothese mit der jeweils gewünschten bzw. erforderlichen, insbesondere nach der Operation exakt gleich bleibenden axialen Länge.

Aus der DE 10 2005 010 705 B3 ist eine Gehörknöchelchenprothese bekannt, bei der eine intraoperative Variabilität der Prothesenlänge dadurch erreicht wird, dass das längliche Verbindungselement in Form einer Kugelkette ausgebildet ist. Diese wird während der Operation mit einer bestimmten Anzahl von Kugeln durch eine Aufnahmeöffnung des ersten Befestigungselements hindurch gesteckt. Danach wird die Kugelkette in der Aufnahmeöffnung des Befestigungselements durch beiderseits der Kugelkette angreifende federnde Stegelemente fixiert und der durch die Aufnahmeöffnung ragende, überstehende Teil der Kugelkette abgezwickt, so dass die Prothese am Ende schließlich genau die gewünschte axiale Länge aufweist. In ähnlicher Weise wird eine Längenvariabilität auch bei einer Gehörknöchelchenprothesen nach der DE 20 2005 015 944 U1 erreicht, wobei hier wiederum eine abzwickbare Kugelkette als Verbindungselement verwendet wird, jedoch die Aufnahme im ersten Befestigungselement anders gestaltet ist.

Eine weitere passive Gehörknöchelchenprothese mit intraoperativ veränderbarer axialer Länge ist in der US-A 3,710,399 beschrieben. Hier wird ein zweigeteiltes Verbindungselement zwischen den beiden Befestigungselementen verwendet, das zwei parallel verlaufende gerade Drahtstücke umfasst, von denen das eine vom ersten und das andere vom zweiten Befestigungselement wegragt. Die beiden Drahtstücke können entweder mittels Drahtschlingen an ihren Enden mit dem jeweils andern Drahtstück verbunden oder in eine Art Verbindungsmuffe mit zwei parallelen Längsbohrungen für die beiden Drahtstücke gesteckt werden. Im ersteren Fall ist jedoch die Fixierungsposition und damit die relative Lage der beiden Drahtstücke nur sehr ungenau einzustellen, so dass eine exakte und reproduzierbare Längeneinstellung der Prothese nicht möglich ist. Im zweiten Fall kann es nach dem Einstecken der Drahtstücke in die Verbindungsmuffe leicht zu Verkippungen, Verknickungen oder Verschiebungen der relativen Lagen der Drahtstücke zueinander kommen, wodurch ebenfalls eine genaue Einstellung der axialen Prothesenlänge erschwert bzw. unmöglich gemacht wird.

Wiederum eine andere Technik der Längeneinstellung wird bei einer passiven Gehörknöchelchenprothese angewendet, wie sie aus der DE 10 2005 027 215 A1 bekannt ist. Diese Prothese zielt ausschließlich auf die Situation einer Steigbügel-Operation ab, so dass immer ein kolbenförmiges Piston als zweites Befestigungselement vorgesehen ist. In diesem Piston ist ein Aufnahmemechanismus angeordnet, in welche das schaftförmige Verbindungselement in axialer Richtung eingeschoben werden soll. Von dem Verbindungselement radial abgespreizte Blattfedern sollen dann in einer gewünschten relativen Position zwischen Verbindungselement und zweitem Befestigungselement eine Arretierung bewirken. Abgesehen davon, dass eine exakt reproduzierbare Einstellung einer gewünschten axialen Länge der Prothese damit nicht immer garantiert sein dürfte, ist der Anwendungsbereich dieser Gehörknöchelchenprothese lediglich auf Steigbügel-Operationen beschränkt, bei der über das Piston eine unmittelbare Verbindung zum Innenohr hergestellt wird. Soll jedoch als zweites Befestigungsteil beispielsweise eine Glocke, ein Stempel, ein Clip oder ein flacher Schuh für eine Verbindung mit einem anderen Teil der Gehörknöchelchenkette verwendet werden, ist diese bekannte Prothese nicht verwendbar. Wenn man nämlich einen entsprechenden Aufnahmemechanismus im zweiten Befestigungsteil unterbringen will, so funktioniert dies schon aus geometrischen Gründen nur in einem Kolben, aber niemals in einer Glocke, einem flachen Schuh oder gar in einem Clip.

Einen ebenfalls stark eingeschränkten Anwendungsbereich deckt die in der DE 297 22 084 U1 beschriebene längenvariable Gehörknöchelchenprothese ab, die anstelle eines schaftförmigen Verbindungselements drei stativartig abknickbare Stegelemente aufweist, welche einenends in einen glockenförmigen oder stempelförmigen Körper zur Befestigung am Steigbügel und anderenends in eine Kopfplatte zur Anlage am Trommelfell münden. Diese Konstruktion kann ausschließlich in Verbindung mit einem plattenartigen Befestigungselement, also lediglich bei Ankopplung am Trommelfell eingesetzt werden. Nachteilig ist bei dieser Prothese außerdem, dass kein definierter Schaft als Verbindungselement zwischen den beiden Befestigungselementen vorhanden ist, so dass es bei nicht völlig exakter axialer Krafteinleitung leicht zu einem Ausweichen bzw. Ausknicken quer zur Längsachse der Prothese kommen kann.

Die in der US-A 5,554,188 beschriebene Gehörknöchelchenprothese umfasst wiederum ein als zweigeteilter Schaft aufgebautes Verbindungselement, bei dem der erste, stangenförmige Abschnitt in eine Aufnahmebohrung des als Aufnahmeteil ausgebildeten zweiten Abschnitts einführbar und axial in der Bohrung verschiebbar ist. Um eine gewünschte axiale Länge der Prothese zu erhalten, wird der stangenförmige erste Abschnitt ausgehend von einer maximalen Ausgangslänge auf eine geeignete Endlänge abgeschnitten und bis auf Anschlag in den zweiten Abschnitt eingeschoben. Durch eine entsprechende Gestaltung des lichten Durchmessers der Aufnahmebohrung relativ zum Außendurchmesser des ersten Abschnitts soll nun eine friktionale Verklemmung von erstem und zweitem Abschnitt eine gewisse Fixierung der Prothesenlänge bewirken, wobei die eigentliche Fixierung dadurch erreicht wird, dass sich die gegeneinander beweglichen Teile der Prothese nach der operativen Einführung ins Mittelohr aufgrund ihres jeweiligen Anschlages an den beiden Befestigungspunkten nicht allzu weit voneinander entfernen können. Eine exakt gleich bleibende Prothesenlänge kann damit allerdings auf Dauer nicht sichergestellt werden.

Bei der in der US 2003/0097178 A1 beschriebenen passiven Gehörknöchelchenprothese weist darüber hinaus das Aufnahmeteil einen in Richtung auf das Einschiebeteil hin offenen Hohlraum auf, der sich in axialer Richtung des Verbindungselements erstreckt, das Verbindungselement ist in axialer Richtung zwischen dem Aufnahmeteil und dem Einschiebeteil Längen variabel gestaltet, und die Festlegung der konkreten axialen Länge des Verbindungselements einer individuellen Gehörknöchelchenprothese erfolgt durch Verklemmen des Einschiebeteils mit dem Aufnahmeteil in einer gewünschten relativen koaxialen Einschiebeposition. Damit könnte im Prinzip eine gewünschte, definierte Länge der Prothese auch schon vor einem Einklemmen zwischen den beiden Befestigungspunkten hergestellt werden, wobei diese Länge auch nach Abschluss der Operation, etwa nach dem Durchstecken eines als Piston ausgebildeten zweiten Befestigungselements durch eine perforierte Steigbügel-Fußplatte, fix beibehalten wird.

Aus der DE 20 2007 012 217 U1 ist eine Gehörknöchelchenprothese bekannt, bei welcher die Klemmkraft zwischen dem Aufnahmeteil und dem Einschiebeteil im verklemmten Zustand erheblich größer gewählt wird als die maximalen, natürlicherweise im Mittelohr im Bereich der Gehörknöchelchen auftretenden äußeren Kräfte. Dies ermöglicht eine Längenvariabilität der passiven Gehörknöchelchenprothese "in situ" bzw. intraoperativ, wobei weder größere Sortimente von Prothesen unterschiedlicher Längen während jeder Operation bereitgehalten werden müssen. Außerdem ist die Einstellung der jeweils gewünschten individuellen Prothesenlänge und damit deren Handhabung besonders einfach. Nachträgliche postoperative unerwünschte Längen- und/oder Lageänderungen der Prothese werden durch die vorgeschriebene Wahl der Klemmkraft sicher vermieden. Zudem ist diese bekannte Gehörknöchelchenprothese universell bei allen denkbaren Arten von Ankoppelungen im Mittelohrraum einsetzbar und nicht auf eine bestimmte Klasse von Operationen beschränkt, während beispielsweise die Prothese gemäß der oben zitierten DE 10 2005 027 215 A1 ausschließlich in der Situation einer Steigbügel-Operation verwendet werden kann. Erkauft werden diese Vorteile allerdings durch einen relativ komplizierten mechanischen Aufbau der Verstellvorrichtung im Verbindungselement der Prothese, der natürlich erheblichen Fertigungsaufwand und damit höhere Herstellungskosten verursacht.

Die eingangs zitierte DE 10 2009 016 468 B3 schließlich offenbart eine bezüglich der vorliegenden Erfindung gattungsgemäße Gehörknöchelchenprothese, bei welcher einerseits die Anzahl der intraoperativ bereit zuhaltenden unterschiedlichen Prothesen ganz erheblich reduziert werden kann, ohne dabei die Möglichkeit zur optimalen Adaption der Prothese im konkreten Einzelfall zu verlieren, andererseits aber die komplexe Konstruktion der Verstellvorrichtung der aus der DE 10 2005 027 215 A1 bekannten Gehörknöchelchenprothese durch einen wesentlich einfacheren mechanischen Aufbau umgangen wird. Dies wird dadurch erreicht, dass die Verstellvorrichtung mindestens zwei symmetrisch zur Längsachse des Verbindungselements verlaufende, in Achsrichtung streckbare und/oder stauchbare Teilstränge umfasst, die dauerhaft bleibend plastisch verformbar und zumindest vor ihrem Verformen quer zur Längsachse in mehreren Schlaufen gefaltet sind.

Die Einstellung dieser bekannten Gehörknöchelchenprothese auf eine bestimmte gewünschte Länge erfolgt intra-operativ durch mechanisches Stauchen oder Auseinanderziehen von einer oder mehreren Schlaufen in der Verstellvorrichtung.

Aufgabe der vorliegenden Erfindung ist es demgegenüber, eine gattungsgemäße längenvariable Mittelohrprothese der eingangs definierten Art mit möglichst einfachen technischen Mitteln unaufwändig und kostengünstig dahin gehend zu verbessern, dass eine Längenverstellung der Gehörknöchelchenprothese intra-operativ auch berührungsfrei erfolgen kann.

Erfindungsgemäß wird diese -bei genauerer Betrachtung relativ komplexe und anspruchsvolle- Aufgabe auf ebenso überraschend einfache wie wirkungsvolle Weise dadurch gelöst, dass die Schlaufen der Verstellvorrichtung aus einem Material mit Formgedächtnis hergestellt sind, dass bei jeder der Schlaufen zumindest eines der beiden Teilstränge an dem von der Längsachse radial am weitesten entfernten Außenbereich jeweils eine Aktivierungszunge mechanisch und wärmeleitend befestigt ist, wobei die Aktivierungszunge radial von der Schlaufe weg ragt und eine senkrecht zur Längsachse orientierte Aktivierungsfläche aufweist, welche durch Hitzeeinwirkung eine thermische Aktivierung der zugehörigen Schlaufe bewirken kann, und wobei die zu den Schlaufen gehörenden Aktivierungsflächen in axialer Richtung vom ersten Befestigungselement entlang der Längsachse des Verbindungselements zum zweiten Befestigungselement hin sukzessive einen immer größeren radialen Abstand von der Längsachse haben, und dass ein Durchbruch durch die perforierte Kopfplatte des ersten Befestigungselement geometrisch so gestaltet ist, dass in Blickrichtung längs der Längsachse auf das erste Befestigungselement sämtliche Aktivierungsflächen durch diesen Durchbruch hindurch optisch sichtbar sind, so dass sie mittels thermischer Strahlung berührungslos von außen her erhitzt werden und dadurch die zugehörigen Schlaufen aktivieren können.

Auf diese Weise kann die erfindungsgemäße Gehörknöchelchenprothese intra-operativ nach ihrem Einsetzen in das Mittelohr des Patienten berührungslos auf optischem Weg durch Einstrahlung von Energie durch den Durchbruch durch die perforierte Kopfplatte hindurch auf die Aktivierungsfläche einer Aktivierungszunge in ihrer axialen Länge angepasst werden, indem die eingestrahlte Energie die entsprechende Aktivierungszunge erhitzt und über Wärmeleitung schließlich die zugehörige Schlaufe der der Verstellvorrichtung thermisch aktiviert wird, die dann -je nach Art und Ausgestaltung des Bereichs aus Material mit Memory Effekt- sich öffnet oder zusammenklappt.

Bei besonders einfach herzustellenden Ausführungsformen der Erfindung sind die Aktivierungszungen in Form von rechteckigen Plättchen mit unterschiedlicher Länge in radialer Richtung zur Längsachse ausgeführt.

Um alle Aktivierungsflächen einfach auf optischem Wege durch den Durchbruch durch die perforierte Kopfplatte hindurch zu erreichen, sind bei vorteilhaften Ausführungsformen der erfindungsgemäßen Gehörknöchelchenprothese die Aktivierungszungen derart angeordnet, dass die sukzessive immer größeren radialen Abstände der Aktivierungsflächen von der Längsachse in axialer Richtung in gleich großen Schritten von einer Schlaufe zur nächstfolgenden inkrementieren.

Ganz besonders bevorzugt sind Ausführungsformen der erfindungsgemäßen Gehörknöchelchenprothese, bei denen mindestens ein, vorzugsweise mehrere quer zur Längsachse verlaufende Verbindungsstege vorgesehen sind, welche jeweils eine Schlaufe eines Teilstrangs mit einer Schlaufe eines parallelen eines Teilstrangs verbinden. Dadurch werden beim Auseinanderziehen oder Stauchen der Verstellvorrichtung die parallelen Teilstränge an bestimmten Stellen jeweils in einer exakt definierten Distanz zueinander gehalten, so dass die Verstellvorrichtung nach ihrer plastischen Verformung eine genau vorgegebbare Geometrie aufweist.

Eine Klasse von Ausführungsformen der erfindungsgemäßen Gehörknöchelchenprothese zeichnet sich dadurch aus, dass die Schlaufen der Verstellvorrichtung im angelieferten Zustand der Prothese eng zusammengefaltet sind, zur Einstellung einer gewünschten axialen Länge der Prothese in Richtung der Längsachse des länglichen Verbindungselements von einem Operateur auseinander gezogen werden können und nach der Implantation der Prothese ins Mittelohr des Patienten in diesem auseinander gezogenen Zustand plastisch verformt verharren.

Bei einer dazu alternativen Klasse von Ausführungsformen sind die Schlaufen der Verstellvorrichtung im angelieferten Zustand der Gehörknöchelchenprothese auseinander gezogen und können zur Einstellung einer gewünschten axialen Länge der Prothese in Richtung der Längsachse des länglichen Verbindungselements von einem Operateur zusammen gestaucht werden. Nach der Implantation der Prothese ins Mittelohr des Patienten verharren sie dann plastisch verformt in diesem zusammengestauchten Zustand.

Um ein gleichmäßiges, definiertes Dehnen bzw. Stauchen der Verstellvorrichtung beim plastischen Verformen zu erzielen, weisen bei einer Klasse von Ausführungsformen die Schlaufen der Verstellvorrichtung quer zur Längsachse des länglichen Verbindungselements jeweils die gleiche maximale Ausdehnung auf.

Bei einer alternativen Klasse von Ausführungsformen weisen axial gegenüberliegende Schlaufenpaare der Verstellvorrichtung quer zur Längsachse des länglichen Verbindungselements unterschiedliche, insbesondere von einem axialen Ende der Verstellvorrichtung zum anderen axialen Ende hin stetig zunehmende oder abnehmende maximale Ausdehnungen auf. Damit kann eine bestimmte Reihenfolge vorgegeben werden, in der die einzelnen Schlaufen sich beim Dehnen bzw. Stauchen der Verstellvorrichtung verformen. Diejenige Schlaufe mit der größten maximalen Ausdehnung wird im Hinblick auf ihre plastische Verformbarkeit in der Regel die weichste sein und sich daher bei Krafteinwirkung als erste verformen.

Bei einer besonders einfachen und kompakten Klasse von Ausführungsformen der erfindungsgemäßen Gehörknöchelchenprothese bildet die Verstellvorrichtung selbst schon das längliche Verbindungselement.

Bei einer dazu alternativen Klasse von Ausführungsformen ist das längliche Verbindungselement -wie üblich- als Schaft gestaltet.

Bevorzugt sind Weiterbildungen dieser Ausführungsformen, bei denen die Verstellvorrichtung in den Schaft integriert und an beiden axialen Enden der Verstellvorrichtung ein Verbindungsstück zum ersten Befestigungselement bzw. zum zweiten Befestigungselement angeordnet ist, was der Prothese in axialer Richtung eine gewisse Formstabilität verleiht.

Ganz besonders vorteilhaft sind Ausführungsformen der erfindungsgemäßen Gehörknöchelchenprothese, bei denen an beiden axialen Enden der Verstellvorrichtung, vorzugsweise mit axialem Abstand zum ersten Befestigungselement bzw. zum zweiten Befestigungselement, jeweils eine Eingriffseinrichtung vorgesehen ist, an welcher mit einem Bedingungsinstrument, beispielsweise einer Pinzette oder Zange, jeweils ein Kraft- oder Formschluss hergestellt werden kann, um die Verstellvorrichtung durch Krafteinwirkung in Richtung der Längsachse des Verbindungselements auseinander zu ziehen oder zu stauchen.

Eine Klasse von Ausführungsformen der erfindungsgemäßen Gehörknöchelchenprothese zeichnet sich dadurch aus, dass das zweite Befestigungselement zur mechanischen Verbindung mit dem Steigbügelkopf oder der Steigbügel-Fußplatte als Hülse, als geschlossene Glocke, als einfach oder mehrfach geschlitzte Glocke oder als Clip ausgebildet ist. Bei Weiterbildungen dieser Ausführungsformen ist die Prothese über eine Kopfplatte einerseits am Trommelfell und über das zweite Befestigungselement andererseits am Amboss oder am Steigbügel befestigt.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen einer Kopfplatte und dem Verbindungselement kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse postoperative Mobilität aufweist, so dass sich die Kopfplatte postoperativ selbstständig der Position des Trommelfells angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

In der Regel wird bei der erfindungsgemäßen GehörknöchelchenProthese das Verbindungselement zwischen den beiden Befestigungselementen als länglicher Schaft gestaltet sein, wie dies an sich aus dem Stand der Technik wohlbekannt ist. Um die oben erörterte erhöhte Flexibilität bzw. Variabilität der Prothese zu erreichen -wie beispielsweise ausführlich in der EP 1 181 907 B1 beschrieben- kann bei einer besonders bevorzugten Ausführungsform der Erfindung im länglichen Verbindungselement mindestens ein Gelenk vorgesehen sein, vorzugsweise ein Kugelgelenk, insbesondere eine axial verlaufende Kugelgelenk-Kette. Vorteilhaft im Hinblick auf eine besonders hohe postoperative Beweglichkeit der Prothese sind Weiterbildungen, bei denen der längliche Schaft eine Vielzahl von aneinander angrenzenden weiteren Drehelementen, vorzugsweise eine Kugelgelenkkette, umfasst.

Möglich sind Ausführungsformen der Erfindung, bei denen die Prothese oder Teile davon aus biokompatiblen Kunststoffen, insbesondere Silikon, Polytetrafluorethylen (PTFE) oder Polyetheretherketon (PEEK), und/oder aus Faserverbundwerkstoffen, insbesondere Kohlefasern hergestellt sind. Mit diesen Materialien können postoperative Abstoßungsreaktionen in den meisten Fällen verhindert werden.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen Teile der Gehörknöchelchenprothese, insbesondere die Schlaufen der Verstellvorrichtung, aus Nitinol hergestellt sind.

Alternativ oder ergänzend können bei weiteren Ausführungsformen Teile der erfindungsgemäßen Gehörknöchelchenprothese aus einem Keramikmaterial hergestellt sein.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager" dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein als Kopfplatte ausgebildetes Befestigungselement sollte bei der erfindungsgemäßen Gehörknöchelchenprothese grundsätzlich eine wachstumsfördernde Beschichtung, andere Teile der Prothese hingegen eine wachstumshemmende Beschichtung aufweisen.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgegebenen oder vorgebbaren Frequenzgang der Schallleitung im Mittelohr berechnet ist. Damit lässt sich ohne großen zusätzlichen technischen Aufwand gewissermaßen ein mechanisches Tuning der Schallfortpflanzungseigenschaften mittels einer individuellen ausgestalteten Gehörknöchelchenprothese erreichen. Ein solcher Tuning-Effekt kann bei speziellen Ausführungsformen beispielsweise dadurch erzielt werden, dass mindestens eine zusätzliche Masse in Abhängigkeit von einem gewünschten, vorgebbaren Frequenz-gang der Schallleitung im Mittelohr an einem Teil der Gehörknöchelchenkette bzw. der Prothese befestigt ist. Bei vorteilhaften Weiter-bildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung schließlich zeichnet sich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Gehörknöchelchenprothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

In den Rahmen der vorliegenden Erfindung fällt auch ein Verfahren zur thermischen Aktivierung der Schlaufen einer Gehörknöchelchenprothese der oben beschriebenen erfindungsgemäßen Art, welches sich dadurch auszeichnet, dass die Aktivierungsfläche einer Aktivierungszunge, deren zugehörige Schlaufe thermischen aktiviert werden soll, durch den Durchbruch im ersten Befestigungselement hindurch mit einem Laserstrahl derart beschossen wird, dass sich die entsprechende Aktivierungsfläche erhitzt. Die Verwendung eines Laserstrahls zur thermischen Aktivierung ermöglicht ein besonders präzises und feines Arbeiten bei der Implantation der Prothese.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. In der schematischen Zeichnung sind Ausführungsbeispiele der Erfindung dargestellt, welche in der nachfolgenden Beschreibung näher erläutert werden.

Im Einzelnen zeigen:
- Fig. 1: eine räumliche Darstellung einer Ausführungsform der erfindungsgemäßen Gehörknöchelchenprothese mit einer perforierten Trommelfell-Kopfplatte als erstem Befestigungselement, einer Verstellvorrichtung mit eng zusammengefalteten Schlaufen von jeweils gleicher Querausdehnung sowie einem stempelförmigen zweiten Befestigungselement;
- Fig. 2: die Ausführungsform nach Fig. 1 in schematischer Ansicht von der Seite; und
- Fig. 3: die perforierte Trommelfell-Kopfplatte der Ausführungsform nach Fig. 1 in axialer Draufsicht von oben durch den Durchbruch auf die Aktivierungsflächen, die in axialer Richtung sukzessive einen immer größeren radialen Abstand von der Längsachse des Verbindungselements haben.

Die in den Figuren der Zeichnung schematisch dargestellte Ausführungsform der erfindungsgemäßen **Gehörknöchelchenprothese 10** weist am einen Ende ein **erstes Befestigungselement 11** auf, welches der mechanischen Verbindung der Prothese mit dem Trommelfell dient und daher als perforierte Kopfplatte zur Anlage am Trommelfell ausgebildet ist. Am anderen Ende der Gehörknöchelchenprothese 10 sitzt ein **zweites Befestigungselement 12** zur mechanischen Verbindung der Prothese mit dem Steigbügelkopf oder der Steigbügel-Fußplatte. Dazwischen ist ein die beiden Befestigungselemente 11 und 12 Schall leitend miteinander verbindendes, längliches **Verbindungselement 13** angeordnet.

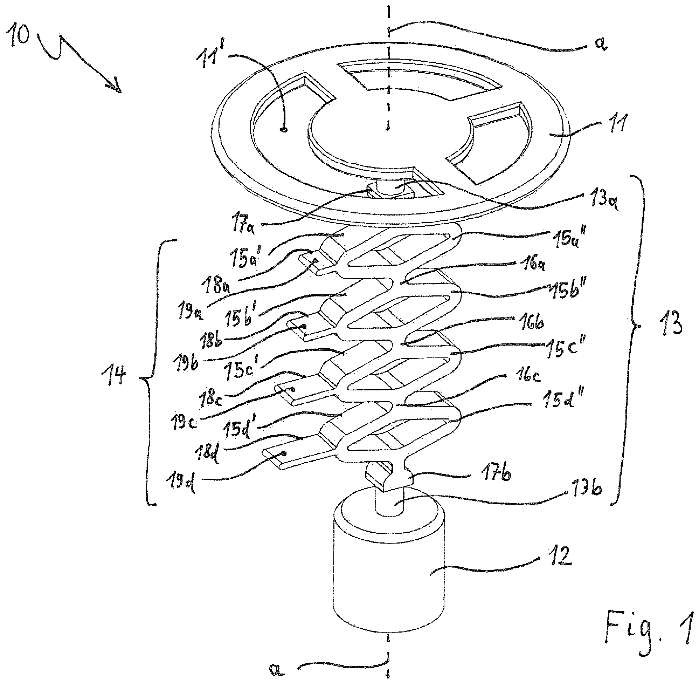

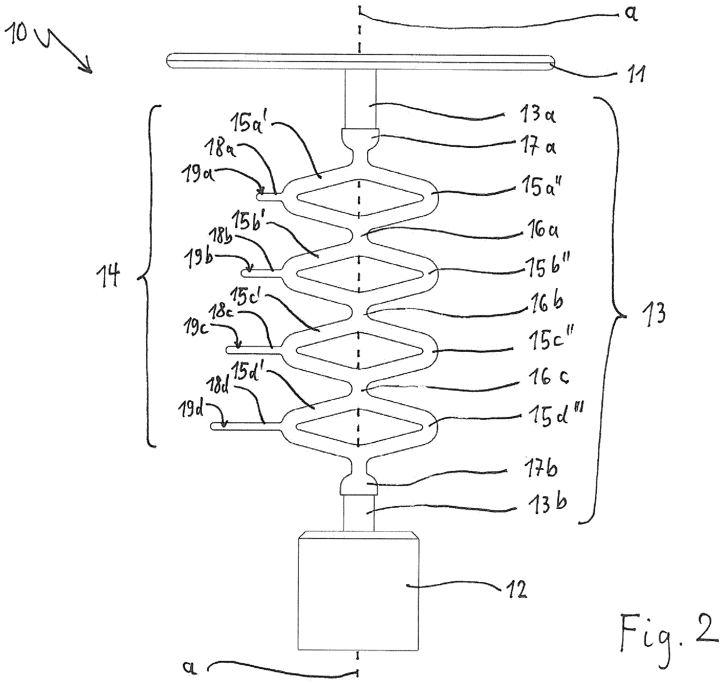

Das zweite Befestigungselement 12 in den Figuren 1 und 2 ist Stempelförmig ausgestaltet. Bei in der Zeichnung nicht dargestellten Ausführungsformen der erfindungsgemäßen Gehörknöchelchenprothese kann das zweite Befestigungselement auch anders gestaltet sein, etwa als geschlitzte Glocke, als Clip, als Hülse, als Schlinge oder als Haken. Auch kann beispielsweise das zweite Befestigungselement in Form einer Klammer ausgebildet sein.

Das Verbindungselement 13 umfasst eine **Verstellvorrichtung 14** zur individuellen Einstellung der axialen Länge der Gehörknöchelchenprothese 10 in Achsrichtung des länglichen Verbindungselements 13, wobei jeweils das erste Befestigungselement 11 mit einem Ende und das zweite Befestigungselement 12 mit dem axial entgegen gesetzten anderen Ende des Verbindungselements 13 mechanisch starr verbunden ist.

Die Verstellvorrichtung 14 umfasst mindestens zwei symmetrisch zur **Längsachse a** des Verbindungselements 13 verlaufende, in Achsrichtung streckbare und/oder stauchbare Teilstränge, die zur Festlegung der axialen Länge der Gehörknöchelchenprothese 10 dauerhaft bleibend plastisch verformbar und vor dem Verformen quer zur Längsachse a in mehreren **Schlaufen 15a',15b',15c',15d, 15a",15b",15c",15d"** Schlangenförmig, Mäanderförmig bzw. Ziehharmonika-artig gefaltet sind.

Jeder Teilstrang umfasst mindestens drei Schlaufen 15a',15b',15c',15d' bzw. 15a",15b",15c",15d", wobei zumindest Teile der Gehörknöchelchenprothese 10 aus einem Material mit Formgedächtnis, wie etwa Nitinol, hergestellt sind.

Gegenüber dem Stand der Technik zeichnet sich die erfindungsgemäße Gehörknöchelchenprothese 10 durch folgende Merkmale aus:

Die Schlaufen 15a',15b',15c',15d', 15a",15b",15c",15d" der Verstellvorrichtung 14 sind sämtlich aus einem Material mit Formgedächtnis hergestellt.

Bei jeder der Schlaufen 15a',15b',15c',15d' zumindest eines der beiden Teilstränge ist an dem von der Längsachse a radial am weitesten entfernten Außenbereich jeweils eine **Aktivierungszunge 18a,18b,18c,18d** mechanisch und wärmeleitend befestigt ist, wobei jede Aktivierungszunge 18a,18b,18c,18d radial von der Schlaufe 15a',15b',15c',15d' weg ragt und eine senkrecht zur Längsachse a orientierte **Aktivierungsfläche 19a,19b,19c,19d** aufweist, welche durch Hitzeeinwirkung eine thermische Aktivierung der zugehörigen Schlaufe 15a',15b',15c',15d' bewirken kann, und wobei die zu den Schlaufen 15a',15b',15c',15d' gehörenden Aktivierungsflächen 19a,19b,19c,19d in axialer Richtung vom ersten Befestigungselement 11 entlang der Längsachse a des Verbindungselements 13 zum zweiten Befestigungselement 12 hin sukzessive einen immer größeren radialen Abstand von der Längsachse a haben, insbesondere in axialer Richtung in gleich großen Schritten von einer Schlaufe 15a',15b',15c',15d' zur nächstfolgenden inkrementierend.

Im vorliegenden Ausführungsbeispiel der Erfindung sind die Aktivierungszungen 18a,18b,18c,18d in Form von rechteckigen Plättchen mit unterschiedlicher Länge in radialer Richtung zur Längsachse a ausgeführt.

Schließlich ist ein **Durchbruch 11'** durch die perforierte Kopfplatte des ersten Befestigungselement 11 geometrisch so gestaltet, dass in Blickrichtung längs der Längsachse a auf das erste Befestigungselement 11 sämtliche Aktivierungsflächen 19a,19b,19c,19d durch diesen Durchbruch 11' hindurch optisch sichtbar sind, so dass sie mittels thermischer Strahlung, vorzugsweise mittels Laser, berührungslos von außen her erhitzt werden und dadurch die zugehörigen Schlaufen 15a',15b',15c',15d' aktivieren können.

Bei der in den Figuren der Zeichnung gezeigten Ausführungsform sind quer zur Längsachse a verlaufende **Verbindungsstege 16a,16b,16c** vorgesehen, welche jeweils eine Schlaufe 15a',15b',15c',15d' eines Teilstrangs mit einer Schlaufe 15a",15b",15c",15d" des parallelen Teilstrangs verbinden.

Bei der in den Figuren 1 und 2 dargestellten Ausführungsform der Erfindung ist an beiden axialen Enden der Verstellvorrichtung 14 mit axialem Abstand zum ersten Befestigungselement 11 bzw. zum zweiten Befestigungselement 12 jeweils eine **Eingriffseinrichtung 17a,17b** vorgesehen ist, an welcher mit einem Bedingungsinstrument, beispielsweise einer Pinzette oder Zange, jeweils ein Kraft- oder Formschluss hergestellt werden kann, um die Verstellvorrichtung 14; zusätzlich durch mechanische Krafteinwirkung in Richtung der Längsachse a des Verbindungselements 13 auseinander zu ziehen oder zu stauchen, etwa in Fällen, wenn keine Laserstrahleinrichtung verfügbar ist.

In der Regel wird das längliche Verbindungselement 13 als zweigeteilter Schaft gestaltet sein, wie in den Figuren der Zeichnung gezeigt. Die Verstellvorrichtung 14 ist hier jeweils in den Schaft integriert. An beiden axialen Enden der Verstellvorrichtung 14 ist ein **Verbindungsstück 13a bzw. 13b** zum ersten Befestigungselement 11 bzw. zum zweiten Befestigungselement 12 angeordnet.

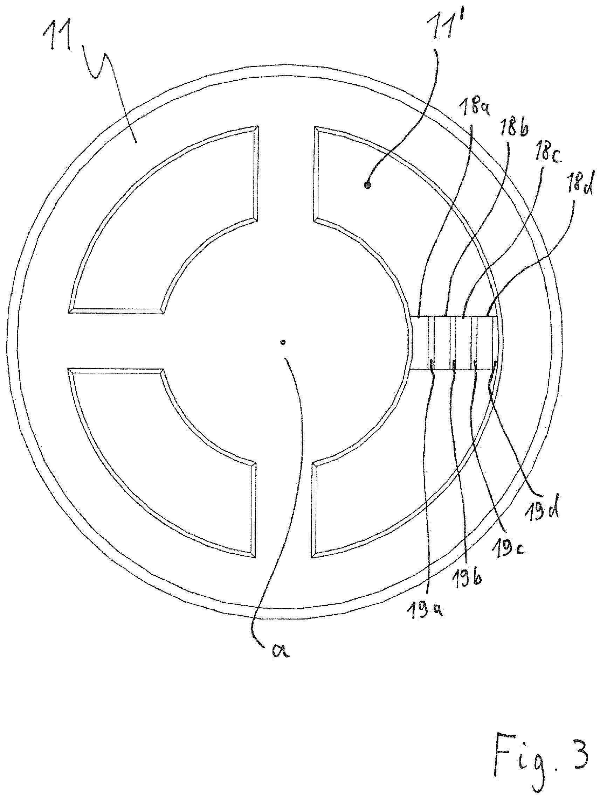

Die Ausführungsform nach den Figuren 1 bis 3 zeichnet sich weiterhin dadurch aus, dass die Schlaufen 15a',15b',15c',15d' sowie 15a",15b",15c",15d" der Verstellvorrichtung 14 quer zur Längsachse a des länglichen Verbindungselements 13 jeweils die gleiche maximale Ausdehnung aufweisen. Bei in der Zeichnung nicht dargestellten Ausführungsformen können axial gegenüberliegende Schlaufenpaare der Verstellvorrichtung quer zur Längsachse a des länglichen Verbindungselements aber auch unterschiedliche, insbesondere vom Kopfplattenseitigen Ende der Verstellvorrichtung zum anderen axialen Ende hin stetig zunehmende maximale Ausdehnungen aufweisen.

Bei der in der Zeichnung dargestellten Ausführungsform der Erfindung handelt es sich um ein passives Implantat. Die erfindungsgemäße Gehörknöchelchenprothese kann aber -bei nicht gezeigten Ausführungsformen- auch als Teil eines aktiven Gehörimplantatsystems ausgestaltet sein.

Zum Handhabung und Bearbeitung der erfindungsgemäßen Gehörknöchelchenprothese während der Implantation ist ein Verfahren zur thermischen Aktivierung der Schlaufen 15a',15b',15c',15d', 15a",15b",15c",15d" besonders geeignet, bei welchem die Aktivierungsfläche 19a,19b,19c,19d einer Aktivierungszunge 18a,18b,18c,18d, deren zugehörige Schlaufe 15a',15b',15c',15d', 15a",15b",15c", 15d" thermischen aktiviert werden soll, durch den Durchbruch 11' im ersten Befestigungselement 11 hindurch mit einem Laserstrahl derart beschossen wird, dass sich die entsprechende Aktivierungsfläche 19a,19b,19c,19d erhitzt

## Patentansprüche

1. Gehörknöchelchenprothese (10), die mindestens ein Glied der menschlichen Gehörknöchelchenkette ersetzt oder überbrückt, wobei die Gehörknöchelchenprothese (10) an einem Ende ein als perforierte Kopfplatte zur Anlage am Trommelfell ausgebildetes erstes Befestigungselement (11) und an ihrem anderen Ende ein zweites Befestigungselement (12) zur mechanischen Verbindung der Prothese mit dem Steigbügelkopf oder der Steigbügel-Fußplatte sowie ein die beiden Befestigungselemente (11 bzw. 12) Schall leitend miteinander verbindendes, längliches Verbindungselement (13) aufweist, welches eine Verstellvorrichtung (14) zur Einstellung der axialen Länge der Gehörknöchelchenprothese (10) in Achsrichtung des länglichen Verbindungselements (13) umfasst, wobei das erste Befestigungselement (11) mit einem Ende und das zweite Befestigungselement (12) mit dem axial entgegengesetzten anderen Ende des Verbindungselements (13) mechanisch starr verbunden ist, wobei die Verstellvorrichtung (14) mindestens zwei symmetrisch zur Längsachse (a) des Verbindungselements (13) verlaufende, in Achsrichtung streckbare und/oder stauchbare Teilstränge umfasst, die dauerhaft bleibend plastisch verformbar und vor dem Verformen quer zur Längsachse (a) in mehreren Schlaufen (15a',15b',15c', 15d', 15a",15b",15c",15d") Schlangenförmig, Mäanderförmig bzw. Ziehharmonika-artig gefaltet sind, wobei jeder Teilstrang mindestens drei Schlaufen (15a',15b',15c',15d' bzw. 15a",15b",15c",15d") umfasst, und wobei Teile der Gehörknöchelchenprothese (10) aus einem Material mit Formgedächtnis hergestellt sind,
**dadurch gekennzeichnet,**
**dass** die Schlaufen (15a',15b',15c',15d', 15a",15b",15c",15d") der Verstellvorrichtung (14) aus einem Material mit Formgedächtnis hergestellt sind,
**dass** bei jeder der Schlaufen (15a',15b',15c',15d') zumindest eines der beiden Teilstränge an dem von der Längsachse (a) radial am weitesten entfernten Außenbereich jeweils eine Aktivierungszunge (18a,18b,18c,18d) mechanisch und wärmeleitend befestigt ist, wobei die Aktivierungszunge (18a,18b,18c,18d) radial von der Schlaufe (15a',15b',15c',15d') weg ragt und eine senkrecht zur Längsachse (a) orientierte Aktivierungsfläche (19a,19b,19c,19d) aufweist, welche durch Hitzeeinwirkung eine thermische Aktivierung der zugehörigen Schlaufe (15a',15b',15c',15d') bewirken kann, und wobei die zu den Schlaufen (15a',15b',15c',15d') gehörenden Aktivierungsflächen (19a,19b,19c,19d) in axialer Richtung vom ersten Befestigungselement (11) entlang der Längsachse (a) des Verbindungselements (13) zum zweiten Befestigungselement (12) hin sukzessive einen immer größeren radialen Abstand von der Längsachse (a) haben,
und **dass** ein Durchbruch (11') durch die perforierte Kopfplatte des ersten Befestigungselement (11) geometrisch so gestaltet ist, dass in Blickrichtung längs der Längsachse (a) auf das erste Befestigungselement (11) sämtliche Aktivierungsflächen (19a,19b,19c,19d) durch diesen Durchbruch (11') hindurch optisch sichtbar sind, so dass sie mittels thermischer Strahlung berührungslos von außen her erhitzt werden und dadurch die zugehörigen Schlaufen (15a',15b',15c',15d') aktivieren können.

2. Gehörknöchelchenprothese nach Anspruch 1, **dadurch gekennzeichnet, dass** die Aktivierungszungen (18a,18b,18c,18d) in Form von rechteckigen Plättchen mit unterschiedlicher Länge in radialer Richtung zur Längsachse (a) ausgeführt sind.

3. Gehörknöchelchenprothese nach einem der vorhergehenden Ansprüche, **dadurch gekennzeichnet, dass** die sukzessive immer größeren radialen Abstände der Aktivierungsflächen (19a,19b,19c,19d) von der Längsachse (a) in axialer Richtung in gleich großen Schritten von einer Schlaufe (15a',15b',15c',15d') zur nächstfolgenden inkrementieren.

4. Gehörknöchelchenprothese nach einem der vorhergehenden Ansprüche, **dadurch gekennzeichnet, dass** quer zur Längsachse (a) verlaufende Verbindungsstege (16a,16b,16c), vorhanden sind, welche jeweils eine Schlaufe (15a',15b',15c', 15d') eines Teilstrangs mit einer Schlaufe (15a",15b",15c",15d") des parallelen Teilstrangs verbinden.

5. Gehörknöchelchenprothese nach einem der Ansprüche 1 bis 4,
**dadurch gekennzeichnet, dass** die Schlaufen (15a',15b',15c',15d', 15a",15b",15c",15d") der Verstellvorrichtung (14) im angelieferten Zustand der Gehörknöchelchenprothese (10) eng zusammengefaltet sind, zur Einstellung einer gewünschten axialen Länge der Prothese in Richtung der Längsachse (a) des länglichen Verbindungselements (13) von einem Operateur auseinander gezogen werden können und nach der Implantation der Prothese ins Mittelohr des Patienten in diesem auseinander gezogenen Zustand plastisch verformt verharren.

6. Gehörknöchelchenprothese nach einem der Ansprüche 1 bis 4,
**dadurch gekennzeichnet, dass** die Schlaufen (15a',15b',15c',15d', 15a",15b",15c",15d") der Verstellvorrichtung (14) im angelieferten Zustand der Gehörknöchelchenprothese (10) auseinander gezogen sind, zur Einstellung einer gewünschten axialen Länge der Prothese in Richtung der Längsachse (a) des länglichen Verbindungselements (13) von einem Operateur zusammen gestaucht werden können und nach der Implantation der Prothese ins Mittelohr des Patienten in diesem zusammengestauchten Zustand plastisch verformt verharren.

7. Gehörknöchelchenprothese nach einem der Ansprüche 1 bis 6,
**dadurch gekennzeichnet, dass** die Schlaufen (15a',15b',15c',15d', 15a",15b",15c",15d") der Verstellvorrichtung (14) quer zur Längsachse (a) des länglichen Verbindungselements (13) jeweils die gleiche maximale Ausdehnung aufweisen.

8. Gehörknöchelchenprothese nach einem der Ansprüche 1 bis 6, **dadurch gekennzeichnet, dass** axial gegenüberliegende Schlaufenpaare der Verstellvorrichtung quer zur Längsachse (a) des länglichen Verbindungselements (13) unterschiedliche maximale Ausdehnungen aufweisen.

9. Gehörknöchelchenprothese nach einem der Ansprüche 1 bis 8,
**dadurch gekennzeichnet, dass** die Verstellvorrichtung das längliche Verbindungselement bildet.

10. Gehörknöchelchenprothese nach einem der Ansprüche 1 bis 8,
**dadurch gekennzeichnet, dass** das längliche Verbindungselement (13) als Schaft gestaltet ist, dass die Verstellvorrichtung (14) in den Schaft integriert ist, und dass an beiden axialen Enden der Verstellvorrichtung (14) ein Verbindungsstück (13a bzw. 13b) zum ersten Befestigungselement (11) bzw. zum zweiten Befestigungselement (12 bzw. 22) angeordnet ist.

11. Gehörknöchelchenprothese nach einem der vorhergehenden Ansprüche, **dadurch gekennzeichnet, dass** das Verbindungselement (13) mindestens ein Gelenk aufweist.

12. Gehörknöchelchenprothese nach einem der vorhergehenden Ansprüche, **dadurch gekennzeichnet, dass** das zweite Befestigungselement (12) zur mechanischen Verbindung mit dem Steigbügelkopf oder der Steigbügel-Fußplatte als Hülse, als geschlossene Glocke, als einfach oder mehrfach geschlitzte Glocke oder als Clip ausgebildet ist.

13. Gehörknöchelchenprothese nach einem der vorhergehenden Ansprüche, **dadurch gekennzeichnet, dass** Teile der Gehörknöchelchenprothese (10) aus Nitinol hergestellt sind.

14. Gehörknöchelchenprothese nach einem der vorhergehenden Ansprüche, **dadurch gekennzeichnet, dass** die Gehörknöchelchenprothese (10) Teil eines aktiven Gehörimplantatsystems ist.

15. Verfahren zur thermischen Aktivierung der Schlaufen (15a',15b',15c',15d', 15a",15b",15c",15d") einer Gehörknöchelchenprothese (10) nach einem der vorhergehenden Ansprüche, **dadurch gekennzeichnet, dass** die Aktivierungsfläche (19a,19b,19c,19d) einer Aktivierungszunge (18a,18b,18c,18d), deren zugehörige Schlaufe (15a',15b',15c',15d', 15a",15b",15c", 15d") thermischen aktiviert werden soll, durch den Durchbruch (11') im ersten Befestigungselement (11) hindurch mit einem Laserstrahl derart beschossen wird, dass sich die entsprechende Aktivierungsfläche (19a,19b,19c,19d) erhitzt.

## Claims

1. Auditory ossicle prosthesis (10), which replaces or bridges at least one member of the human ossicular chain, in which at one end the auditory ossicle prosthesis (10) has a first fastening element (11) made as a perforated head plate for lying on the ear drum and at its other end a second fastening element (12) for mechanical connection of the prosthesis to the stapes head or the stapes footplate as well as an elongated connecting element (13) connecting both fastening elements (11 or 12) to each other in a sound-conducting way, which comprises an adjustment device (14) for adjusting the axial length of the auditory ossicle prosthesis (10) in the axial direction of the elongated connecting element (13), in which the first fastening element (11) is connected to one end and the second fastening element (12) to the axially opposite other end of the connecting element (13) in a mechanically rigid way, in which the adjustment device (14) comprises at least two part sections that may be extended and/or compressed in the axial direction running symmetrically to the longitudinal axis (a) of the connecting element (13), which may be plastically deformed on a permanent basis and before being deformed are folded into several serpentine, meandering or concertina type loops (15a', 15b', 15c', 15d', 15a", 15b", 15c", 15d") perpendicular to the longitudinal axis (a), in which each part section comprises at least three loops (15a', 15b', 15c', 15d' or 15a", 15b", 15c", 15d") and in which parts of the auditory ossicle prosthesis (10) are produced from a material having memory effect, **characterised in that,**
the loops (15a', 15b', 15c', 15d', 15a", 15b", 15c", 15d") of the adjustment device (14) are produced from a material having memory effect,
in each of the loops (15a', 15b', 15c', 15d') of at least one of both part sections an activating tongue (18a, 18b, 18c, 18d) is fastened mechanically in a heat-conducting way to the outside area that is radially furthest away from the longitudinal axis (a), in which the activating tongue (18a, 18b, 18c, 18d) projects radially away from the loop (15a', 15b', 15c', 15d') and has an activating area (19a, 19b, 19c, 19d) that is orientated perpendicular to the longitudinal axis (a), which may cause thermal activation of the relevant loops (15a', 15b', 15c', 15d') through the effect of heat and in which the activating areas (19a, 19b, 19c, 19d) belonging to the loops (15a', 15b', 15c', 15d') successively have an increasingly greater radial distance from the longitudinal axis (a) in the axial direction from the first fastening element (11) along the longitudinal axis (a) of the connecting element (13) to the second fastening element (12),
and an opening (11') through the perforated head plate of the first fastening element (11) is made geometrically so that all the activating areas (19a, 19b, 19c, 19d) on the first fastening element (11) are visible optically through this opening (11') in the direction of view along the longitudinal axis (a), so that they may be heated from outside without contact by means of thermal radiation and may activate the relevant loops (15a', 15b', 15c', 15d') through this.

2. Auditory ossicle prosthesis according to claim 1, **characterised in that** the activating tongues (18a, 18b, 18c, 18d) are made in the form of rectangular plates of differing length in the radial direction to the longitudinal axis (a).

3. Auditory ossicle prosthesis according to one of the previous claims, **characterised in that** the successive increasingly greater radial distances of the activating areas (19a, 19b, 19c, 19d) from the longitudinal axis (a) in the axial direction increase in steps of the same size from one loop (15a', 15b', 15c', 15d') to the next.

4. Auditory ossicle prosthesis according to one of the previous claims, **characterised in that** there are connecting bridges (16a, 16b, 16c) running perpendicular to the longitudinal axis (a), which each connect a loop (15a', 15b', 15c', 15d') of a part section to a loop (15a", 15b", 15c", 15d") of the parallel part section.

5. Auditory ossicle prosthesis according to one of claims 1 to 4, **characterised in that** the loops (15a', 15b', 15c', 15d', 15a", 15b", 15c", 15d") of the adjustment device (14) are folded closely together in the delivered state of the auditory ossicle prosthesis (10), may be pulled apart by a surgeon for adjusting to a desired axial length of the prosthesis in the direction of the longitudinal axis (a) of the elongated connecting element (13) and remain plastically deformed in this pulled apart state after the prosthesis is implanted in the middle ear of the patient.

6. Auditory ossicle prosthesis according to one of claims 1 to 4, **characterised in that** the loops (15a', 15b', 15c', 15d', 15a", 15b", 15c", 15d") of the adjustment device (14) are pulled apart in the delivered state of the auditory ossicle prosthesis (10), may be pressed together by a surgeon for adjusting to a desired axial length of the prosthesis in the direction of the longitudinal axis (a) of the elongated connecting element (13) and remain plastically deformed in this pressed together state after the prosthesis is implanted in the middle ear of the patient.

7. Auditory ossicle prosthesis according to one of claims 1 to 6, **characterised in that** the loops (15a', 15b', 15c', 15d', 15a", 15b", 15c", 15d") of the adjustment device (14) each have the same maximum extent perpendicular to the longitudinal axis (a) of the elongated connecting element (13).

8. Auditory ossicle prosthesis according to one of claims 1 to 6, **characterised in that** axially opposite pairs of loops of the adjustment device have different maximum extents perpendicular to the longitudinal axis (a) of the elongated connecting element (13).

9. Auditory ossicle prosthesis according to one of claims 1 to 8, **characterised in that** the adjustment device forms the elongated connecting element.

10. Auditory ossicle prosthesis according to one of claims 1 to 8, **characterised in that** the elongated connecting element (13) is made as a shaft, the adjustment device (14) is integrated into the shaft and a connecting piece (13a or 13b) for the first fastening element (11) or the second fastening element (12 or 22) is arranged at both axial ends of the adjustment device (14).

11. Auditory ossicle prosthesis according to one of the previous claims, **characterised in that** the connecting element (13) has at least one joint.

12. Auditory ossicle prosthesis according to one of the previous claims, **characterised in that** the second fastening element (12) for mechanical connection to the stapes head or the stapes footplate is made as a sleeve, a closed bell, a bell with one or several slots or a clip.

13. Auditory ossicle prosthesis according to one of the previous claims, **characterised in that** parts of the auditory ossicle prosthesis (10) are produced from nitinol.

14. Auditory ossicle prosthesis according to one of the previous claims, **characterised in that** the auditory ossicle prosthesis (10) is part of an active auditory implant system.

15. Method for thermally activating the loops (15a', 15b', 15c', 15d', 15a", 15b", 15c", 15d") of an auditory ossicle prosthesis (10) according to one of the previous claims, **characterised in that** the activating areas (19a, 19b, 19c, 19d) of an activating tongue (18a, 18b, 18c, 18d), the loops belonging to which (15a', 15b', 15c', 15d', 15a",15b",15c", 15d") are to be thermally activated, are fired at with a laser beam through the opening (11') in the first fastening element (11) in such a way that the corresponding activating area (19a, 19b, 19c, 19d) heats up

## Revendications

1. Prothèse d'osselet auditif (10) qui remplace ou ponte au moins un élément de la chaîne d'osselets humaine, la prothèse d'osselet (10) comportant à une extrémité un premier élément de fixation (11) qui est configuré sous la forme d'une plaque de tête perforée destinée à l'appui contre le tympan ou à son autre extrémité un second élément de fixation (12) destiné à la liaison mécanique de la prothèse avec la tête de l'étrier ou avec la platine de l'étrier, ainsi qu'un élément raccord (13) allongé destiné à la liaison conductrice du son des deux éléments de fixation (11 ou 12), qui comprend un dispositif de déplacement (14) pour régler la longueur axiale de la prothèse d'osselet (10) en direction axiale de l'élément raccord allongé (13),
dans laquelle
le premier élément de fixation (11) est relié de façon mécaniquement rigide à une extrémité et le second élément de fixation (12) est relié de façon mécaniquement rigide à l'autre extrémité axialement opposée de l'élément raccord (13),
le dispositif de déplacement (14) comprend au moins deux brins partiels étirables et/ou comprimables en direction axiale qui s'étendent symétriquement à l'axe longitudinal (a) de l'élément raccord (13) et qui sont déformables plastiquement de façon durable et permanente et qui sont pliés, avant la déformation, transversalement à l'axe longitudinal (a) en plusieurs boucles (15a', 15b', 15c', 15d', 15a", 15b", 15c", 15d") en forme de serpentin, de méandres ou d'accordéon,
chaque brin partiel comprend au moins trois boucles (15a', 15b', 15c', 15d' ou 15a", 15b", 15c", 15d"), et
des parties de la prothèse d'osselet (10) sont réalisées en un matériau à mémoire de forme,
**caractérisée en ce que**
les boucles (15a', 15b', 15c', 15d', 15a", 15b", 15c", 15d") du dispositif de déplacement (14) sont réalisées en un matériau à mémoire de forme,
**en ce que** pour chacune des boucles (15a', 15b', 15c', 15d') de l'un au moins des brins partiels, une languette d'activation respective (18a, 18b, 18c, 18d) est fixée mécaniquement et de façon thermoconductrice sur la zone extérieure radialement la plus éloignée de l'axe longitudinal (a),
la languette d'activation respective (18a, 18b, 18c, 18d) fait saillie radialement en éloignement de la boucle (15a', 15b', 15c', 15d') et présente une surface d'activation (19a, 19b, 19c, 19d) orientée perpendiculairement à l'axe longitudinal (a) qui est apte à provoquer par effet de chaleur une activation thermique de la boucle associée (15a', 15b', 15c', 15d'),
et les surfaces d'activation (19a, 19b, 19c, 19d) faisant partie des boucles (15a', 15b', 15c', 15d') présentent une distance radiale successivement de plus en plus importante depuis l'axe longitudinal (a) en direction axiale depuis le premier élément de fixation (11) le long de l'axe longitudinal (a) de l'élément raccord (13) vers le second élément de fixation (12),
et **en ce qu'**une traversée (11') à travers la plaque de tête perforée du premier élément de fixation (11) est configurée géométriquement de telle sorte qu'en direction d'observation le long de l'axe longitudinal (a) vers le premier élément de fixation (11), toutes les surfaces d'activation (19a, 19b, 19c, 19d) sont visibles optiquement à travers cette traversée (11'), de manière à pouvoir être chauffées sans contact depuis l'extérieur par rayonnement thermique et à pouvoir ainsi activer les boucles associées (15a', 15b', 15c', 15d').

2. Prothèse d'osselet selon la revendication 1, **caractérisée en ce que** les languettes d'activation (18a, 18b, 18c, 18d) sont réalisées sous forme de platines rectangulaires de différentes longueurs en direction radiale par rapport à l'axe longitudinal (a).

3. Prothèse d'osselet selon l'une des revendications précédentes,
**caractérisée en ce que** les distances radiales successivement de plus en plus importantes depuis l'axe longitudinal (a) des surfaces d'activation (19a, 19b, 19c, 19d) en direction axiale augmentent par incrément à des pas égaux d'une boucle (15a', 15b', 15c', 15d') à l'autre.

4. Prothèse d'osselet selon l'une des revendications précédentes,
**caractérisée en ce qu'**il existe des barrettes de liaison (16a, 16b, 16c) qui s'étendent transversalement à l'axe longitudinal (a) qui relient chacune une boucle (15a', 15b', 15c', 15d') d'un brin partiel à une boucle (15a", 15b", 15c", 15d") de l'autre brin partiel parallèle.

5. Prothèse d'osselet selon l'une des revendications 1 à 4, **caractérisée en ce qu'**à l'état de livraison de la prothèse d'osselet (10), les boucles (15a', 15b', 15c', 15d', 15a", 15b", 15c", 15d") du dispositif de déplacement (14) sont étroitement repliées, et pour régler une longueur axiale désirée de la prothèse en direction de l'axe longitudinal (a) de l'élément raccord allongé (13), elles peuvent être étirées par un opérateur, et une fois que la prothèse est implantée dans l'oreille moyenne du patient, elles demeurent plastiquement déformées dans cet état étiré.

6. Prothèse d'osselet selon l'une des revendications 1 à 4, **caractérisée en ce qu'**à l'état de livraison de la prothèse d'osselet (10), les boucles (15a', 15b', 15c', 15d', 15a", 15b", 15c", 15d") du dispositif de déplacement (14) sont étirées, et pour régler une longueur axiale désirée de la prothèse en direction de l'axe longitudinal (a) de l'élément raccord allongé (13), elles peuvent être comprimées par un opérateur, et une fois que la prothèse est implantée dans l'oreille moyenne du patient, elles demeurent plastiquement déformées dans cet état comprimé.

7. Prothèse d'osselet selon l'une des revendications 1 à 6, caractérisée en que les boucles (15a', 15b', 15c', 15d', 15a", 15b", 15c", 15d") du dispositif de déplacement (14) présentent chacune la même extension maximale transversalement à l'axe longitudinal (a) de l'élément raccord allongé (13).

8. Prothèse d'osselet selon l'une des revendications 1 à 6, **caractérisée en ce que** des paires de boucles axialement opposées du dispositif de déplacement présentent différentes extensions maximales transversalement à l'axe longitudinal (a) de l'élément raccord allongé (13).

9. Prothèse d'osselet selon l'une des revendications 1 à 8, **caractérisée en ce que** le dispositif de déplacement constitue l'élément raccord allongé.

10. Prothèse d'osselet selon l'une des revendications 1 à 8, **caractérisée en ce que** l'élément raccord allongé (13) est réalisé sous forme de tige, **en ce que** le dispositif de déplacement (14) est intégré dans la tige, et **en ce qu'**un organe de liaison (13a ou 13b) vers le premier élément de fixation (11) ou vers le second élément de fixation (12 ou 22) est agencé respectivement aux deux extrémités axiales du dispositif de déplacement (14).

11. Prothèse d'osselet selon l'une des revendications précédentes,
**caractérisée en ce que** l'élément raccord (13) comprend au moins une articulation.

12. Prothèse d'osselet selon l'une des revendications précédentes,
**caractérisée en ce que** le second élément de fixation (12) destiné à la liaison mécanique avec la tête de l'étrier ou la platine de l'étrier est réalisé sous forme de douille, de cloche fermée, de cloche à fente simple ou multiple ou sous forme de clip.

13. Prothèse d'osselet selon l'une des revendications précédentes,
**caractérisée en ce que** des parties de la prothèse d'osselet (10) sont réalisées en nitinol.

14. Prothèse d'osselet selon l'une des revendications précédentes,
**caractérisée en ce que** la prothèse d'osselet (10) fait partie d'un système d'implant auditif actif.

15. Procédé d'activation thermique des boucles (15a', 15b', 15c', 15d', 15a", 15b", 15c", 15d") d'une prothèse d'osselet auditifs (10) selon l'une des revendications précédentes, **caractérisé en ce que** la surface d'activation (19a, 19b, 19c, 19d) d'une languette d'activation (18a, 18b, 18c, 18d), dont la boucle associée (15a', 15b', 15c', 15d', 15a", 15b", 15c", 15d") doit être activée thermiquement est attaquée par un faisceau laser à travers la traversée (11') dans le premier élément de fixation (11) de telle sorte que la surface d'activation correspondante (19a, 19b, 19c, 19d) s'échauffe.
